# EUROPÄISCHE PATENTANMELDUNG

(11) **EP 1 955 591 A1**
(43) Veröffentlichungstag der Anmeldung: **13.08.2008**
(21) Anmeldenummer: 08000218.1
(22) Anmeldetag: 08.01.2008
(51) Int. Cl.: A01N 33/12, A01P 1/00

(54) **Mittel zur Pflege und antimikrobiellen Behandlung der Haut**

(30) Priorität: 09.01.2007 DE 102007002073
(71) Anmelder: Painless Tech GbR, 31134 Hildesheim (DE)
(72) Erfinder: Habenicht, Matthias, 31137 Hildesheim (DE); Zühlsdorff, Marcel, 31141 Hildesheim (DE)
(74) Vertreter: Eisenführ, Speiser & Partner

(57) **Zusammenfassung**

Das Mittel zur Pflege und antimikrobiellen Behandlung der Haut ist einerseits zur Hautdesinfektion im medizinischen und klinischen Bereich und andererseits für die antimikrobielle Haut- und Wundbehandlung geeignet. Dabei sollte das Mittel für die verschiedenen Anwendungsgebiete geeignet, hautpflegend, gut verträglich und desinfizierend sein. Es wurde gefunden, dass diese Aufgabe besonders gut durch Benzalkoniumchlorid als Desinfektionsmittel und antimikrobielles Hautbehandlungsmittel in einer Cremegrundlage erfüllt wird. Das Mittel enthält daher einen Gehalt an Benzalkoniumchlorid in einer für topisch-dermale Applikation geeigneten Grundlage, bei welchem Benzalkoniumchlorid der einzige pharmazeutisch wirksame Bestandteil oder der Hauptwirkbestandteil in der Grundlage ist.

## Beschreibung

Die Erfindung betrifft ein Mittel zur Pflege und antimikrobiellen Behandlung der Haut mit einem Gehalt an Benzalkoniumchlorid in einer für topisch-dermale Applikation geeigneten Grundlage.

Allgemein betrifft die Erfindung das Gebiet der topischen antimikrobiellen Zusammensetzungen mit einer möglichst einige Zeit anhaltenden antiseptischen Wirkung. Derartige Zusammensetzungen können für verschiedene Zwecke verwendet werden. Ein mögliches Anwendungsgebiet betrifft die Verwendung als Hautdesinfektionsmittel im medizinischen bzw. klinischen Bereich. Eine weitere Anwendungsmöglichkeit liegt in der antimikrobiellen Haut- und Wundbehandlung.

Für die Hautdesinfektion, insbesondere von Ärzten oder medizinischem Personal, sind zahlreiche Mittel bekannt. Diese Mittel sind in der Regel flüssig, wodurch sie schnell und einfach aufgetragen werden können und sich gut verteilen. Häufig trocknen sie jedoch die Haut aus. Da der am häufigsten betroffene Personenkreis die Mittel ständig verwenden muss, besteht hier ein Bedürfnis an besser hautverträglichen oder sogar pflegenden Produkten.

Weiterhin werden antiseptische und antimikrobielle Mittel auch zur Behandlung von geschädigter Haut eingesetzt. Hierbei wird in der Regel auf die Selbstheilungskräfte des Körpers vertraut, und man hält gleichzeitig die Wunde oder den geschädigten Bereich von Bakterien frei, um Superinfektionen zu verhindern. Ein Beispiel für derartige Mittel sind Jodsalben, die in verschiedenen Zusammensetzungen im Gebrauch sind. Jodsalben sind für den genannten Zweck besonders bewährt, haben jedoch den Nachteil, dass sie bei Schilddrüsenerkrankungen problematisch sind.

Die Aufgabe der Erfindung bestand daher darin, ein Mittel zur Pflege und antimikrobiellen Behandlung der Haut anzugeben, das für die verschiedenen Anwendungsgebiete geeignet ist und die Nachteile im Stand der Technik vermeidet, d.h. hautpflegend, gut verträglich und desinfizierend ist.

Diese Aufgabe wird gelöst mit Hilfe eines Mittels zur Pflege und antimikrobiellen Behandlung der Haut mit einem Gehalt an Benzalkoniumchlorid in einer für topisch-dermale Applikation geeigneten Grundlage, bei welchem das Benzalkoniumchlorid der einzige pharmazeutisch wirksame Bestandteil oder der Hauptwirkbestandteil in der Grundlage ist.

Im Rahmen der Erfindung wurde gefunden, dass gerade Bezalkoniumchlorid, dessen antiseptische Wirkung grundsätzlich seit langem bekannt ist, innerhalb einer pflegenden Zubereitung für die topische Anwendung, d.h. die .Anwendung auf der Haut, einschließlich der Schleimhaut und des Zahnfleisches, sehr gut pflegend und desinfizierend wirkt. Auf gesunder, wie geschädigter Haut ist Benzalkoniumchlorid gut verträglich. Durch den leicht ansäuernden Charakter des Benzalkoniumchlorids in den Grundzubereitungen liegt der pH-Wert des Mittels in einem für den Säureschutzmantel der Haut optimalen Bereich, so dass diese Mittel sehr pflegend sind und dauerhaft für die Hauthygiene und als Hautdesinfektionsmittel im medizinischen Bereich angewendet werden können. Dies war bislang so noch nicht erkannt worden. Vielmehr wird Benzalkoniumchlorid derzeit hauptsächlich als Konservierungsmittel eingesetzt. Außerdem in einigen speziellen pharmazeutischen Applikationen als Antiseptikum. Die dort erkannte leicht ätzende Wirkung kann jedoch gerade innerhalb einer Salben- oder Cremegrundlage gut ausgeglichen werden, so dass die erfindungsgemäßen Mittel für die Lösung der gestellten Aufgabe überraschenderweise optimal sind.

Ein zusätzlicher Vorteil der Erfindung liegt darin, dass die Mittel universell anwendbar sind. Die gleiche Creme oder Salbe kann einerseits als mildes, pflegendes Desinfektionsmittel auf gesunder Haut, wie auch als Wundbehandlungsmittel, z.B. bei Schürf- oder Brandwunden, bei Druckstellen, bei Akne, oder anderen Dermatosen angewendet werden.

Benzalkoniumchlorid ist ein Gemisch von Alkylbenzyldimethylammonium-chloriden. Der Wirkstoff ist für seine desinfizierende bzw. antiseptische und konservierende Wirkung bekannt. Es wird daher in Desinfektionsmitteln und zur Konservierung von Augentropfen verwendet.

Zwar ist Benzalkoniumchlorid auch innerhalb äußerlich anzuwendender, topischer Mittel bekannt, allerdings nur als zusätzliches antiseptisches Mittel oder Konservierungsmittel neben einem anderen Hauptwirkstoff in untergeordneter Rolle.

So ist beispielsweise aus der DD 232 819 A5 eine pharmazeutische topische Zubereitung bekannt, die zur Förderung der Wundgranulation und Epithelisation insbesondere Calziumsalze enthält. Benzalkoniumchlorid ist daneben lediglich als zusätzlicher Hilfsstoff, speziell als Konservierungsmittel vorgesehen.

Die DE 699 23 695 T2 offenbart topisch-dermale antimikrobielle Zusammensetzungen, die als Wirkstoff ein in Wasser unlösliches Addukt eines hydrophoben polykationischen organischen Polymers mit einem hydrophoben Mittel enthalten. Benzalkoniumchlorid ist dort in einer Reihe mit verschiedenen anderen zusätzlichen antiseptischen Mitteln erwähnt, die ggf. in untergeordneter Menge und Funktion mitverwendet werden können.

Aus der DE 699 21 058 T2 ist ebenfalls ein topisches Mittel bekannt, welches Triclosan enthält und zusätzlich hierzu ein kationisches antibakterielles Mittel aufweisen kann, ausgewählt aus einer Reihe von Stoffen unter anderem auch Benzalkoniumchlorid.

In allen diesen Fällen wurden die bei der Erfindung durch Verwendung allein von Benzalkoniumchlorid in einer für topische Applikation geeigneten Grundlage erzielten Vorteile nicht erkannt.

Grundsätzlich ist vorgesehen, dass das Benzalkoniumchlorid der einzige Wirkstoff innerhalb der pflegenden, vorzugsweise hypoallergenen Grundlage für topische Anwendung ist, denn die erfindungsgemäßen Eigenschaften werden der konkreten Kombination von Benzalkoniumchlorid und pflegender Grundlage zugeschrieben. Weiterhin ist es hauptsächlich vorgesehen, dass das Benzalkoniumchlorid der einzige bzw wesentliche desinfizierende oder antiseptische Wirkstoff des Mittels ist. In Weiterbildung der Erfindung ist es jedoch möglich, dass in der Grundlage eine Mischung pharmazeutischer und/oder desodorierender und/oder desinfizierender Wirkstoffe vorhanden ist, in der der Anteil an Benzalkoniumchlorid größer oder gleich 50 Gew.-% ist. Dies bedeutet, dass das Benzalkoniumchlorid weiterhin als Hauptwirkbestandteil anzusehen ist.

Innerhalb des erfindungsgemäßen Mittels kann der Gehalt an Benzalkoniumchlorid oder der vorgenannten Mischung größer gleich 0,01 Gew.-%, vorzugsweise größer gleich 0,1 Gew.-%, weiter vorzugsweise größer gleich 1 Gew.-% oder größer gleich 3 Gew.-% sein, bezogen auf die Gesamtmasse des Mittels. Er sollte jedoch kleiner 10 Gew.-%, vorzugsweise kleiner 5 Gew.-% sein, um die Verträglichkeit besser zu gewährleisten.

Die Grundlage für die topische Applikation (oder die Grundmasse der Zubereitung) kann vorzugsweise aus einer Salben-, Creme-, Lotion-, Gel-, Suppositorien- oder Schaumgrundlage bestehen, z. B. solchen, wie sie gebrauchsfertig im Handel sind. Insbesondere ist als Grundlage für die topische Applikation jede nicht flüssige Form des Mittels anzusehen, d.h. alle Applikationsformen auf der Haut, außer Benzalkoniumchlorid in Lösung (wie es z. B. für die Desinfektion von Tischen und dergleichen bekannt ist) und insbesondere keine rein wässrige oder alkoholische Applikation ohne Beteiligung von Fett oder Öl.

### Applikationsformen:

Für eine Salbe nach der Erfindung kann die Grundlage für topische Applikation vorzugsweise dickflüssiges bis festes Paraffin, Wollwachs und/oder weißes Vaselin enthalten. Diese Grundstoffe können innerhalb der Grundlage insbesondere in eine Öl-in-Wasser- oder in eine Wasser-in-Öl-Emulsion eingebunden sein, wobei die Grundlage in besonders bevorzugter Ausführungsform aus einer Basishautcreme oder Basislotion in Form einer ÖI-in-Wasser-Emulsion oder einer Wasser-in-Öl-Emulsion bestehen oder diese enthalten kann.

Grundsätzlich versteht man unter einer Salbe eine äußerlich anzuwendende Arzneimittelzubereitung aus einer schmierfähigen oder streichfähigen Grundlage (die man auch als Grundmasse bezeichnen kann) und den auf die Haut aufzubringenden Arzneistoffen, die sich in der Salbe in Lösung oder Suspension befinden können. Häufig handelt es sich bei den Salben um Wasser-in-ÖI-Emülsionen mit einem hohen Fettanteil und einem geringeren Wasseranteil. Als fettige Bestandteile kommen zum Beispiel die folgenden Stoffe einzeln oder im Gemisch in Frage: gelbes Bienenwachs (Cera flava), weißes Bienenwachs (Cera alba), Wollfett (Adeps Lanae, Lanolin, Wollwachsalkohole, Eucerin), Hartfett (Adeps solidus), Vaselin, pflanzliche Öle, wie z.B. Kakaobutter (Oleum Cacao), Erdnussöl, Palmöl, davon abstammende Stoffe, wie z. B. Cetylpalmitat, Cetylstearylalkohol usw., Paraffine und viele andere mehr. Salben können zur Verdickung auch anorganische, pulverförmig eingebrachte Feststoffe enthalten, wie z.B. Zinkoxid.

Den Salben ähnlich sind die Pasten, die i.a. einen höheren Anteil an Feststoffen, z.B. Zinkoxid enthalten.

Unter einer Creme versteht man im medizinischen Sinne eine Öl-in-Wasser-Emulsion oder Wasser-in-Öl-Emulsion, die Inhaltsstoffe applikationsfähig macht. Cremes sind relativ zu den Salben niedrigviskoser, d.h. flüssiger und ziehen besser in die Haut ein. Cremes benötigen häufig Emulgatoren oder Emulsionsstabilisatoren. Allgemein bezeichnen Cremes auch nicht-medizinische rein hautpflegende Produkte, so dass eine Überlappung zu Salben besteht. Unter Lotionen werden hier noch niedriger viskose, fließfähige Cremes verstanden. Lotionen lassen sich mit Gas aufschäumen. Schäume können dazu dienen, einen sparsamen, dünnen und gleichmäßige Auftrag auf der Haut zu erzielen.

Weitere, hier nicht genannte kosmetische oder pharmazeutische Anwendungsformen kommen als Grundlage für die Erfindung ebenfalls in Frage. Allgemein ist von der Erfindung all das erfasst, was zur Anwendung auf der Haut einschließlich von Schleimhaut und Gingiva geeignet ist, einschließlich spezieller Anwendungsformen, wie Gele, insbesondere Hydrogele zur Anwendung auf der Haut, der Schleimhaut oder dem Zahnfleisch. Für Anwendungen im Analbereich kommen zusätzlich Suppositorien in Frage. Schließlich könnten Lotionen oder andere flüssige Applikationsformen auch aufgesprüht werden (Sprays)

Allen Applikationsformen können in untergeordneter Menge andere Stoffe, z.B. Geruchs- oder Geschmacksstoffe (Parfümierung, Aromatisierung), Vitamine, Antioxidantien oder dergleichen enthalten sein. Konservierungsmittel sind wegen des Benzalkoniumgehaltes nicht erforderlich, jedoch auch nicht ausgeschlossen. Alle Zubereitungen sollten hautpflegend und hypoallergen sein.

### Anwendungsgebiete:

Das Mittel ist zur pflegenden Desinfektion der Haut geeignet (Ärzte, medizinisches Personal).

Weiterhin ist das Mittel zur antimikrobiellen Behandlung, kranker, angegriffener oder wunder Haut geeignet, d.h. zur therapeutischen Haut- und Wundbehandlung. Hier umfasste Anwendungen sind: die Verwendung als Antiseptikum auf gesunder oder angegriffener Haut, bei geschädigter Haut, wie z. B. Decubitus (Druckgeschwür), bei Ulcus cruris (Unterschenkelgeschwür), oberflächlichen Wunden, z.B. Schürfwunden und Verbrennungen, infizierten und superinfizierten Dermatosen (Hauterkrankungen aller Art, einschließlich allergischer Erscheinungen, Neurodermitis und Schuppenflechte), Akne. Insbesondere ist eine therapeutische Behandlung jede nicht nur desinfizierende, sondern durch das Mittel auf die Haut wirkende Behandlung.

### BEISPIELE

Das erfindungsgemäße Mittel kann beispielsweise hergestellt werden, indem eine übliche Salben-, Creme-, Lotion- oder Schaumgrundlage mit Benzalkoniumchlorid versetzt wird. Dem Fachmann auf dem pharmazeutischen oder kosmetischen Gebiet sind zahlreiche Beispiele für derartige Grundlagen bekannt, so dass sie hier nicht im Einzelnen beschrieben zu werden brauchen.

Eine geeignete Salbe besteht beispielsweise aus je 25 Gewichtsteilen Paraffinum durum, Adeps lanae, Adeps solidus und Cera flava zuzüglich des gewünschten Gewichtsteils Benzalkoniumchlorid.

Für die nachfolgend angegebenen Hautverträglichkeits- und Anwendungstests wurde eine handelsübliche Grundmasse mit den jeweiligen Benzalkoniumchloridmengen versetzt.

Anhand einer Verdünnungsreihe wurde zunächst festgestellt, welche Konzentrationen an Benzalkoniumchlorid gür die gewünschten Anwendungen günstig sind. Die verwendeten Testkeime waren Escherichia-Coli und Bacillus Subtilis Standard Nährmedium bestehend aus Pepton aus Fleisch; Pepton aus Casein (je 3,45 g/l); Natriumchlorid 5,1 g/l; Agar-Agar 13 g/l. Die Tests wurden in Kulturschalen durchgeführt. Anhand von Vorversuchen mit wässriger Benzalkoniumchloridlösung konnte ermittelt werden, dass innerhalb eines grundsätzlich desinfizierenden Bereichs zwischen 0,0001 und 1 Gew.-% BAC eine besonders gute Wirksamkeit bei 0,1 bis 1 Gew.-%, bevorzugt zwischen 0,1 und 0,5 Gew.-% und besonders bevorzugt um 0,3 Gew.-% erreicht werden kann.

### Hautverträglichkeitstests

untersuchtes Mittel: 0,3 Gew.-% Benzalkoniumchlord in Eucerinum anhydricum Grundlage (Beiersdorf)

Je eine erbsengroße Menge der oben genannten Benzalkoniumcreme wurde 1 mal täglich in den Ellenbogenbeugen je einer Versuchsperson mit normaler Haut und einer Versuchsperson mit empfindlicher Haut aufgetragen. Der Versuch wurde 30 Tage lang durchgeführt. Es trat absolut keine Reizung auf, die Haut war geschmeidig und sehr gut gepflegt.

Das Mittel kann daher auf gesunder Haut über längere Zeiträume gefahrlos angewendet werden und ist als pflegendes Hautdesinfektionsmittel geeignet.

### Behandlung einer Brandblase

Das oben untersuchte Mittel wurde auf eine Verbrennungsstelle am Arm aufgetragen (Verbrennung 1. bis 2. Grades). Die Haut war zu Beginn des Versuchs dunkel gerötet und leicht aufgeworfen. Bereits nach 2 Anwendungstagen war eine deutliche Verbesserung des Hautbildes festzustellen. Nach 7 Tagen war die Verbrennungsstelle vollständig abgeheilt.

### Behandlung einer Akne im Schläfenbereich

Das oben untersuchte Mittel wurde auf pustelige Akne aufgetragen. Bereits nach 4 Tagen waren die Pusteln (Eiterbläschen) abgeheilt. Nach 7 Tagen war eine deutliche Verbesserung des Aknebildes erkennbar.

### Behandlung einer Druckstelle am Fuß

Eine durch Schuhwerk entstandene Druckstelle am Fuß besserte sich spürbar schneller als üblich und war nach 2 Tagen behoben.

## Patentansprüche

1. Mittel zur Pflege und antimikrobiellen Behandlung der Haut mit einem Gehalt an Benzalkoniumchlorid in einer für topisch-dermale Applikation geeigneten Grundlage, **dadurch gekennzeichnet, dass** das Benzalkoniumchlorid der einzige pharmazeutisch wirksame Bestandteil oder der Hauptwirkbestandteil in der Grundlage ist.

2. Mittel nach Anspruch 1, **dadurch gekennzeichnet, dass** in der Grundlage eine Mischung pharmazeutischer und/oder desodorierender und/oder desinfizierender Wirkstoffe vorhanden ist, in der der Anteil an Benzalkoniumchlorid größer oder gleich 50 Gew.-% ist.

3. Mittel nach Anspruch 1 oder 2, **dadurch gekennzeichnet, dass** der Gehalt an Benzalkoniumchlorid oder der vorgenannten Mischung größer gleich 0,01 Gew.-%, vorzugsweise größer gleich 0,1 Gew.-%, weiter vorzugsweise größer gleich 1 Gew.-% oder größer gleich 3 Gew.-% ist, bezogen auf die Gesamtmasse des Mittels.

4. Mittel nach einem der Ansprüche 1 bis 3, **dadurch gekennzeichnet, dass** der Gehalt an Benzalkoniumchlorid oder der oben genannten Mischung kleiner 10 Gew.-%, vorzugsweise kleiner 5 Gew.-% ist.

5. Mittel nach einem der Ansprüche 1 bis 4, **dadurch gekennzeichnet, dass** die Grundlage für topische Applikation aus einer Salben-, Creme-, Lotion-, oder Schaumgrundlage besteht.

6. Mittel nach einem der Ansprüche 1 bis 5, **dadurch gekennzeichnet, dass** die Grundlage für topische Applikation dickflüssiges bis festes Paraffin, Wollwachs, weißes Vaselin, enthält.

7. Mittel nach einem der Ansprüche 1 bis 6, **dadurch gekennzeichnet, dass** die Grundlage für topische Applikation eine Basishautcreme oder Basislotion in Form einer Öl-in-Wasser-Emulsion oder einer Wasser-in-Öl-Emulsion enthält.

8. Mittel nach einem der Ansprüche 1 bis 7, **dadurch gekennzeichnet, dass** es für die therapeutische Haut- und Wundbehandlung bestimmt ist.
